# EUROPEAN PATENT APPLICATION

(11) **EP 2 708 257 A1**
(43) Date of publication of application: **19.03.2014**
(21) Application number: 13182891.5
(22) Date of filing: 03.09.2013
(51) Int. Cl.: A61M 16/04

(54) **Tracheal tube for secretion removal**

(30) Priority: 12.09.2012 TW 101133290
(71) Applicant: Chi Mei Medical Center, Tainan City 710 (TW)
(72) Inventor: Su, Ying-Chieh, 704 Tainan City (TW)
(74) Representative: UEXKÜLL & STOLBERG

(57) **Abstract**

A tracheal tube includes an airway tubular body (31), an inflatable cuff (41) disposed on the airway tubular body (31), a suction conduit (5) extending along the airway tubular body to terminate at a suction opening (52), and a guiding unit (30) disposed on the airway tubular body (31) and adjacent to the suction opening (52) . The guiding unit (30) includes at least one guiding member (301) having a plurality of ribs (302). Each two adjacent ones of the ribs (302) define a guiding groove (303) that extends toward the suction opening (52) and that terminates at a confluent region (304) in fluid communication with the suction opening (52) so as to guide secretions in the trachea (801) to the suction opening (52).

## Description

This disclosure relates to a tracheal tube, more particularly to a tracheal tube including a suction conduit therein.

A conventional cuffed tracheal tube for establishing an airway in a patient is intubated into a trachea of the patient through a mouth, a nose or a tracheostomy stoma of the patient in use. The tracheal tube includes an inflatable cuff which is inflatable to seal against a tracheal wall of the trachea. However, secretions derived from saliva and mucus in the trachea will collect above the cuff, which may cause infections. Another conventional cuffed tracheal tube has a suction opening disposed above the inflatable cuff for suctioning the secretions collecting above the cuff so as to reduce the risk of chest infections in long-term intubated patients. However, when a vacuum is applied to suction the secretions through the suction opening, the vacuum may cause the tracheal wall mucosa membrane to be drawn into the suction opening, which may result in injury to the tracheal wall and obstruct removal of the secretions.

US 2010/0258134 A1 discloses a surgical/medical tube, such as an endotracheal tube, which has a tubular body equipped with an inflatable cuff and separate cuff inflation and fluid removal lumens. A fluid removal opening communicates with the fluid removal lumen and is located proximal to the cuff. A projection is located in close proximity to the fluid removal opening and is configured to prevent contact between the fluid removal opening and the adjacent tracheal wall. Although the provision of the projection may prevent injury to the tracheal wall of the patient during suctioning of the secretions, the secretions still cannot be effectively removed through the fluid removal opening, since the projection may be brought to abut against the tracheal wall by the suction force to obstruct the suctioning of the secretions.

Therefore, an object of the present disclosure is to provide a tracheal tube having a guiding unit which can guide secretions in a trachea of a patient to a suction opening to permit effective removal of the secretions from the trachea of the patient.

Accordingly, a tracheal tube of this disclosure includes an airway tubular body, an inflatable cuff, a suction conduit, and a guiding unit. The airway tubular body extends along a lengthwise axis to terminate at an upstream gas inlet end and a downstream gas outlet end. The downstream gas outlet end is suitable for placement in a trachea of a patient. The upstream gas inlet end is suitable for placement at a region adjacent to an exterior of the patient. The inflatable cuff is mounted on the airway tubular body proximal to and upstream of the downstream gas outlet end, and is inflatable to seal against a tracheal wall of the trachea. The suction conduit extends along the airway tubular body toward the inflatable cuff to terminate at a suction opening which is disposed at a position upstream of the inflatable cuff so as to permit suction of secretions in the trachea therethrough. The guiding unit is disposed on the outer surface of the airway tubular body adjacent to the suction opening, and includes at least one guiding member. The guiding member includes a plurality of ribs. Each two adjacent ones of the ribs define a guiding groove which extends along the lengthwise axis toward the suction opening and which terminates at a confluent region that is in fluid communication with the suction opening so as to guide the secretions in the trachea to the suction opening.

Other features and advantages of the present disclosure will become apparent in the following detailed description of the preferred embodiments of the disclosure, with reference to the accompanying drawings, in which:
Fig. 1 is a perspective view of the first preferred embodiment of a tracheal tube according to this disclosure;
Fig. 2 is a fragmentary cross-sectional view of Fig. 1;
Fig. 3 is a cross-sectional view taken along line III-III of Fig. 1;
Fig. 4 shows the tracheal tube of Fig. 1 in a state of use;
Fig. 5 is a perspective view of the second preferred embodiment of a tracheal tube according to this disclosure; and
Fig. 6 is a fragmentary cross-sectional view of Fig. 5.

Before the present disclosure is described in greater detail, it should be noted herein that same reference numerals are used to denote like elements throughout the specification.

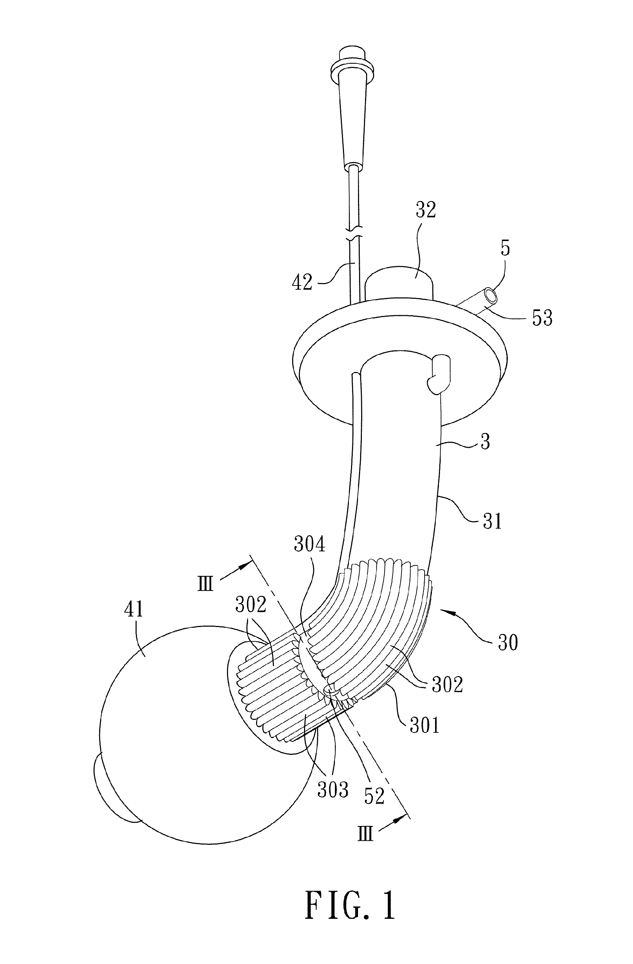

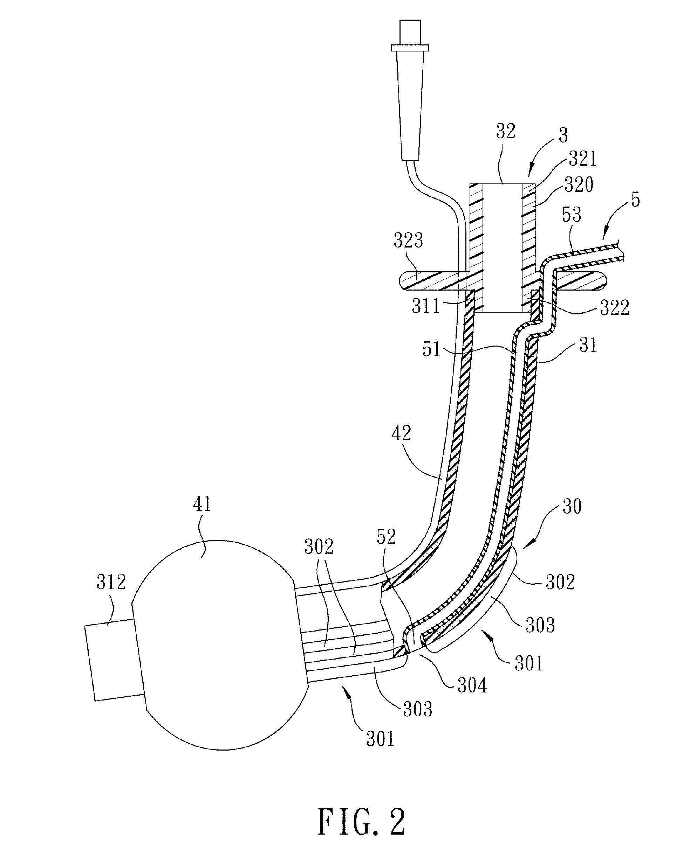

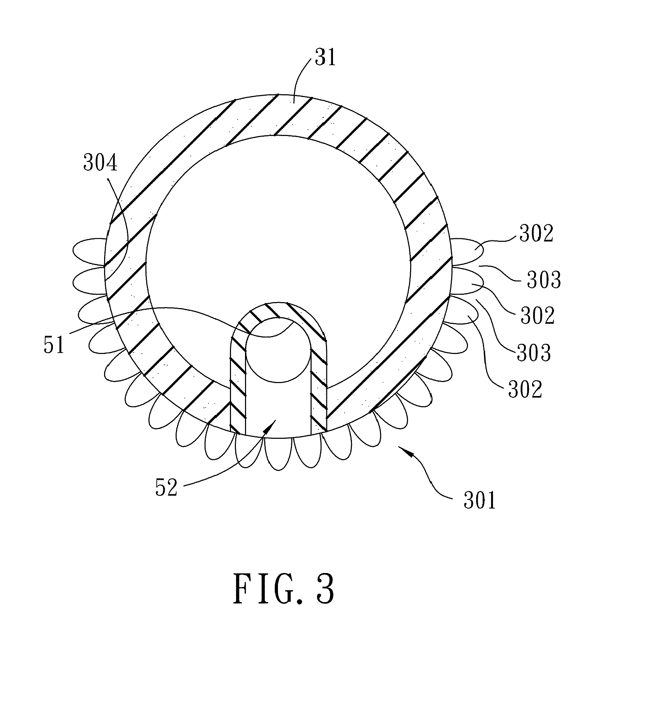

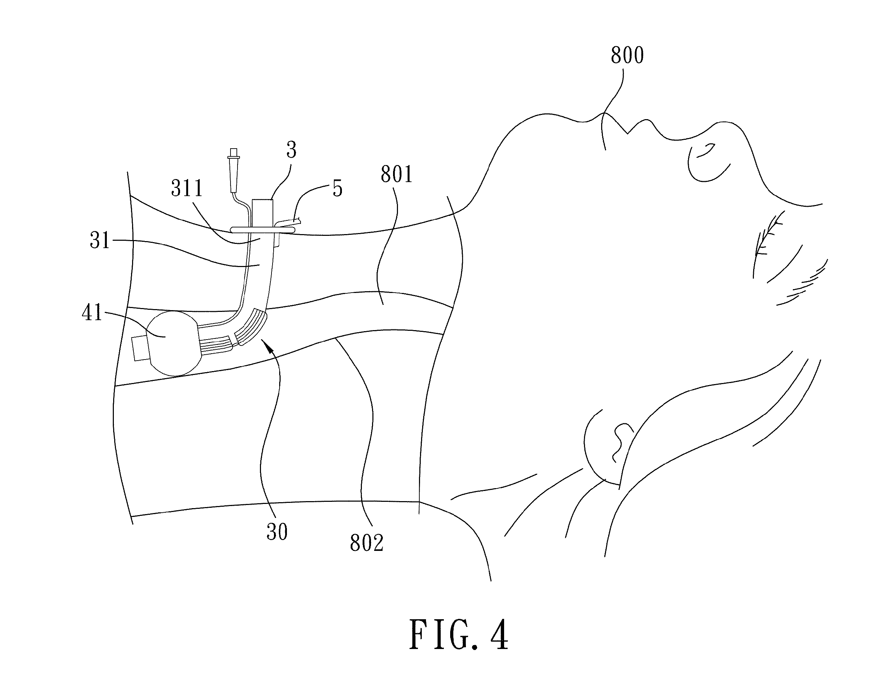

Figs. 1 to 4 illustrate the first preferred embodiment of a tracheal tube 3 according to this disclosure. The tracheal tube 3 is a tracheostomy tube which is adapted to be intubated into a trachea 801 of a patient 800 through a tracheostomy stoma. In other preferred embodiments, the tracheal tube can be an endotracheal tube.

The tracheal tube 3 includes an airway tubular body 31, an inflatable cuff 41, a suction conduit 5, and a guiding unit 30.

The airway tubular body 31 has a curved "L" shape, and extends along a lengthwise axis to terminate at an upstream gas inlet end 311 and a downstream gas outlet end 312. The downstream gas outlet end 312 is suitable for placement in the trachea 801 of the patient 800, and the upstream gas inlet end 311 is suitable for placement at a region adjacent to an exterior of the patient 800, such as the mouth, nose or neck of the patient 800.

The inflatable cuff 41 is mounted on the airway tubular body 31 proximal to and upstream of the downstream gas outlet end 312, and is inflatable to seal against a tracheal wall 802 of the trachea 801. In this embodiment, the tracheal tube 3 further includes an inflating conduit 42 which extends along the outer surface of the airway tubular body 31 and into the inflatable cuff 41 to permit inflation of the inflatable cuff 41 such that the inflatable cuff 41 seals against the tracheal wall 802. Alternately, the inflatable cuff 41 can be inflated by any other known methods.

The suction conduit 5 extends along the airway tubular body 31 toward the inflatable cuff 41 to terminate at a suction opening 52 which is disposed at a position upstream of the inflatable cuff 41 so as to permit suction of secretions in the trachea 801 therethrough.

The suction conduit 5 has two opposite openings, one of which is the suction opening 52, the other of which is a connection opening (not shown) for connection to a suction machine (not shown) which is used to provide a reduced pressure in the trachea 801. In this preferred embodiment, the suction conduit 5 includes an inner segment 51 having the suction opening 52, and an outer segment 53 having the connection opening. The inner segment 51 extends along an inner surface of the airway tubular body 31 and terminates at the suction opening 52 which opens at the outer surface of the airway tubular body 31. The outer segment 53 extends from the inner segment 51 through the airway tubular body 31 and outwardly of the airway tubular body 31.

In another preferred embodiment, the inner segment 51 of the suction conduit 5 may extend between the inner and outer surfaces of the airway tubular body 31.

In yet another preferred embodiment, the suction conduit 5 may extend along the outer surface of the airway tubular body 31.

The guiding unit 30 is disposed on the outer surface of the airway tubular body 31 adjacent to the suction opening 52, and includes at least one guiding member 301. The guiding member 301 includes a plurality of ribs 302, each two adjacent ones of which define a guiding groove 303 that extends along the lengthwise axis toward the suction opening 52 and that terminates at a confluent region 304 that is in fluid communication with the suction opening 52 so as to guide the secretions in the trachea 801 to the suction opening 52. The ribs 302 extend radially and outwardly from the outer surface of the airway tubular body 31 and are disposed to be located adjacent to the tracheal wall 802 so as to prevent the tracheal wall 802 from being engaged in the confluent region 304. In addition, the ribs 302 are displaced from one another in a circumferential direction about the lengthwise axis of the airway tubular body 31.

It should be noted that although, in the preferred embodiment, the guiding groove 303 extends along the lengthwise axis, it can extends in any direction as long as the secretions can be guided by the guiding groove 303 to the suction opening 52.

In this preferred embodiment, the guiding unit 30 includes two guiding members 301 which cooperatively define the confluent region 304 therebetween. One of the guiding members 301 is disposed upstream of the confluent region 304, and the other of the guiding members 301 is disposed downstream of the confluent region 304.

When there is an accumulation of the secretions above the cuff 41, the suction machine can be operated to remove the secretions through the suction opening 52. With the provision of the guiding unit 30, even when the guiding members 31 contact the tracheal wall 801 when the inflatable cuff 41 is inflated, the secretions can still be suctioned through the guiding grooves 303. Furthermore, even if the suction force is unduly strong so that some of the guiding grooves 303 are blocked by tracheal tissue, the secretions can still be suctioned through the guiding grooves 303 that are not blocked.

Preferably, the tracheal tube 3 further includes a tubular connector 32 which includes a tubular stem 320 and a flange 323. The tubular stem 320 has an outer connecting end 321 which is disposed to extend outwardly of the patient 800 so as to be connected to a mechanical ventilator (not shown), and an inner connecting end 322 which is connected to the upstream gas inlet end 311. The flange 323 extends outwardly and radially from an outer surface of the tubular stem 320, and is disposed between the outer and inner connecting ends 321, 322.

In this embodiment, the outer segment 53 of the suction conduit 5 extends along the outer surface of the airway tubular body 31 and through the flange 323 so as to be led out of the patient 800. The inflating conduit 42 is displaced from the suction conduit 5 and also extends through the flange 323 so as to be led out of the patient 800.

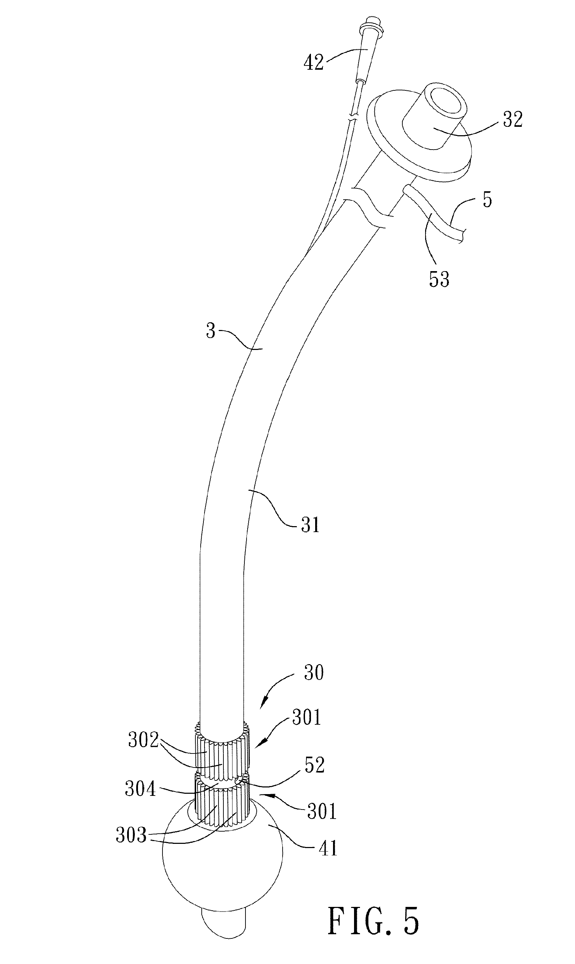

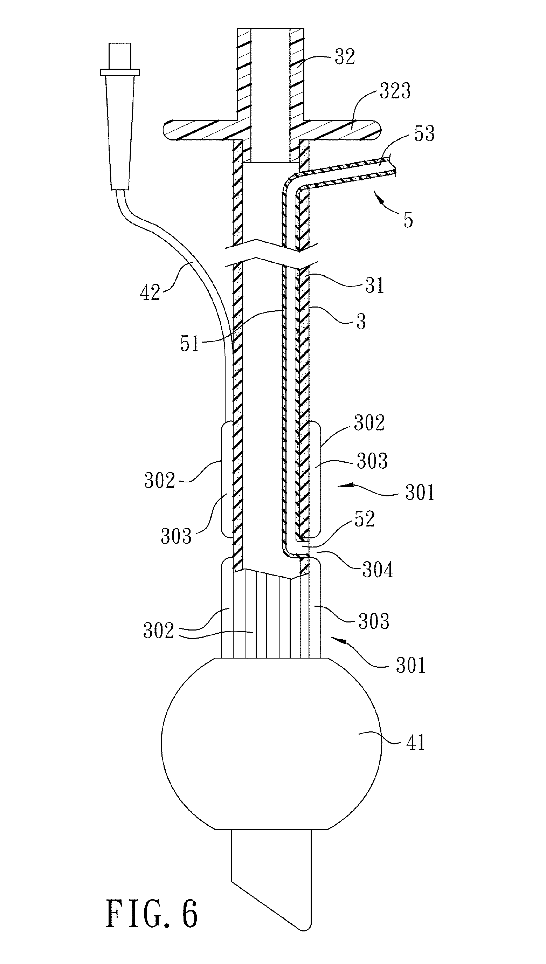

Figs. 5 and 6 show the second preferred embodiment of a tracheal tube according to this disclosure. The tracheal tube of this embodiment is similar to that of the first preferred embodiment, except that, in the second preferred embodiment, the ribs 302 of each guiding member 301 are displaced from one another in a circumferential direction around the airway tubular body 31 such that each guiding member 301 is configured to have a ring shape. Thus, the secretions collecting around the airway tubular body 31 can be suctioned out more efficiently.

In addition, the suction conduit 5 and the inflating conduit 42 do not extend through the flange 323.

## Claims

1. A tracheal tube comprising:
an airway tubular body (31) extending along a lengthwise axis to terminate at an upstream gas inlet end (311) and a downstream gas outlet end (312), said downstream gas outlet end (312) being suitable for placement in a trachea (801) of a patient (800), said upstream gas inlet end (311) being suitable for placement at a region adjacent to an exterior of the patient (800);
an inflatable cuff (41) which is mounted on said airway tubular body (31) proximal to and upstream of said downstream gas outlet end (312), and which is inflatable to seal against a tracheal wall (802) of the trachea (801);
a suction conduit (5) extending along said airway tubular body (31) toward said inflatable cuff (41) to terminate at a suction opening (52) which is disposed at a position upstream of said inflatable cuff (41) so as to permit suction of secretions in the trachea (801) therethrough; and
a guiding unit (30) disposed on said outer surface of said airway tubular body (31) adjacent to said suction opening (52), and including at least one guiding member (301), said guiding member (301) including a plurality of ribs (302), each two adjacent ones of said ribs defining a guiding groove (303) which extends toward said suction opening (52) and which terminates at a confluent region (304) that is in fluid communication with said suction opening (52) so as to guide the secretions in the trachea (801) to said suction opening (52).

2. The tracheal tube of Claim 1, wherein said ribs (302) extend radially and outwardly from said outer surface of said airway tubular body (31) and are disposed to be located adjacent to the tracheal wall (802) so as to prevent the tracheal wall (802) from being engaged in said confluent region (304).

3. The tracheal tube of Claim 1, wherein said guiding unit (30) includes two of said guiding members (301), which cooperatively define said confluent region (304) therebetween, one of said guiding members (301) being disposed upstream of said confluent region (304), the other of said guiding members (301) being disposed downstream of said confluent region (304).

4. The tracheal tube of Claim 1, wherein said ribs (302) are displaced from one another in a circumferential direction about the lengthwise axis.

5. The tracheal tube of Claim 1, wherein said ribs (302) are displaced from one another in a circumferential direction around said airway tubular body (31).

6. The tracheal tube of Claim 1, wherein said suction conduit (5) includes an inner segment (51) extending along an inner surface of said airway tubular body (31) and terminating at said suction opening (52) which opens at said outer surface of said airway tubular body (31).

7. The tracheal tube of Claim 6, further comprising an inflating conduit (42) extending along said outer surface of said airway tubular body (31) and into said inflatable cuff (41) to permit inflation of said inflatable cuff (41) such that said inflatable cuff seals against the tracheal wall (802).

8. The tracheal tube of Claim 7, further comprising a tubular connector (32) which includes:
a tubular stem (320) having an outer connecting end (321) which is disposed to extend outwardly of the patient (800), and an inner connecting end (322) connected to said upstream gas inlet end (311); and
a flange (323) which extends outwardly and radially from an outer surface of said tubular stem (320) and which is disposed between said outer and inner connecting ends (321, 322).

9. The tracheal tube of Claim 8, wherein said suction conduit (5) further includes an outer segment (53) which extends from said inner segment (51) through said airway tubular body (31) and outwardly of said airway tubular body (31).

10. The tracheal tube of Claim 9, wherein said outer segment (53) further extends along said outer surface of said airway tubular body (31) and through said flange (323) so as to be led out of the patient (800).

11. The tracheal tube of Claim 8, wherein said inflating conduit (42) further extends through said flange (323) so as to be led out of the patient (800).

12. The tracheal tube of Claim 1, wherein said guiding groove extends along the lengthwise axis.
